# EUROPEAN PATENT APPLICATION

(11) **EP 0 683 465 A2**
(43) Date of publication of application: **22.11.1995**
(21) Application number: 95303254.7
(22) Date of filing: 16.05.1995
(51) Int. Cl.: G06F 17/60

(54) **Remote access medical network**

(30) Priority: 19.05.1994 US 248267
(71) Applicant: NCR International, Inc., Dayton, Ohio 45479 (US)
(72) Inventor: Yaker, Rhoda, Annandale, New Jersey 08801 (US); Laughlin, David Paul, Kettering, Ohio 45429 (US); Horst, William Richard, (US)
(74) Representative: Robinson, Robert George

(57) **Abstract**

A remote access medical network (10) which minimizes paperwork in connection with health care services includes an insurance network (12) for processing an insurance claim. A remote terminal (30, 34, 38, 42) located at a health care service provider sends the insurance claim to the insurance network. A card reader (32, 36, 40, 44) coupled to the remote terminal (30, 34, 38, 40) reads information about a patient from a patient health care card (50) for completing the insurance claim, and reads payment information from the health care card (50) for completing payment to the health care service provider. The patient health care card (50) is preferably a smart card (50). The smart card (50) may be used to provide payment to the service provider. Insurance payments are obtained by transmitting the insurance claim to the insurance network (12). A financial network (13) credits a bank account of the service provider via an electronic funds transfer.

## Description

The present invention relates to medical information systems and more specifically to a remote access medical network.

Health care providers produce a vast amount of paperwork. Forms must be completed by a patient before seeing a physician. Forms must be completed by physicians, nurses, lab technicians, and many other people for each patient.

Insurance companies are also responsible for a large amount of this paperwork. Insurance companies require forms for completing the process of paying for health care services.

The patient bears the costs associated with the large amount of paperwork required by the health care and insurance industries. One part of the cost includes the cost of non-medical administrative personnel which must be hired to process the paperwork.

U.S. Patent No. 4,491,725 discusses a medical claim verification and processing system wherein a local service provider enters into a local terminal medical and other information and transmits this to a central brokerage computer which determines an insurance claim payment. The amount of such payment is transmitted back to the entry terminal for use by the service provider and patient The service provider can then prepare an electronic clain form which is transmitted via a central brokerage computer to an insurance carrier. The insurance carrier then processes the claim form and arranges for a payment cheque to be sent to the patient or for an electronic funds transfer payment to be made to the patient or direct to the service provider. This known system is complex and has the disadvantage that time is utilized by the insurance carrier in processing the insurance claim.

It is an object of the present invention to provide a simple data processing network for processing medical transactions wherein rapid claim processing is achievable.

Therefore, according to the present invention, there is provided a data processing network for processing medical transactions, characterized by an insurance claim processing netowrk for processing an insurance claim; a remote terminal located at a health care service provider for sending said insurance claim to said insurance claim processing network; and a card reader coupled to said remote terminal, adapted to reaad information about a patient from a card for use in said insurance claim.

Preferably, the insurance network includes a server, and a database coupled to the server for storing the information about a patient. A remote terminal located at a health care service provider sends the insurance claim to the server within the insurance network. A card reader coupled to the remote terminal reads information about a patient from a patient health care card for completing the insurance claim, and reads payment information from the health care card for completing payment to the health care service provider. A financial network credits the account of the service provider via an electronic funds transfer from the insurance company.

The patient health care card is preferably a SMART card. Thus, information about the service provided by the service provider may also be stored in the SMART card and accessed by another health care service provider. For example, if first health care service provider is a physician's office and the second health care service provider is a pharmacy, a prescription from the physician may be stored in the SMART card by the remote terminal at the physician's office and read by the remote terminal at the pharmacy.

Advantageously, no paper is required for providing and filling the prescription. Furthermore, no paper is also required if the SMART card also is used to provide payment to both the physician's office and the pharmacy. A co-payment or deductible is provided through the SMART card.

In order to further reduce paperwork, the remote access medical network also may include an electronic writing tablet coupled to the physician's remote access terminal for recording the prescription, as well as other service information, and a database coupled to the physician's remote access terminal for storing the service information. A scanner may be used to convert paper documents into electronic information for storage in the database.

One embodiment of the invention will now be described by way of example with reference to the accompanying drawings, in which:
Fig. 1 is a block diagram of the remote access medical network of the present invention; and
Fig. 2 is a flow chart illustrating the operation of the remote access medical network of the present invention.

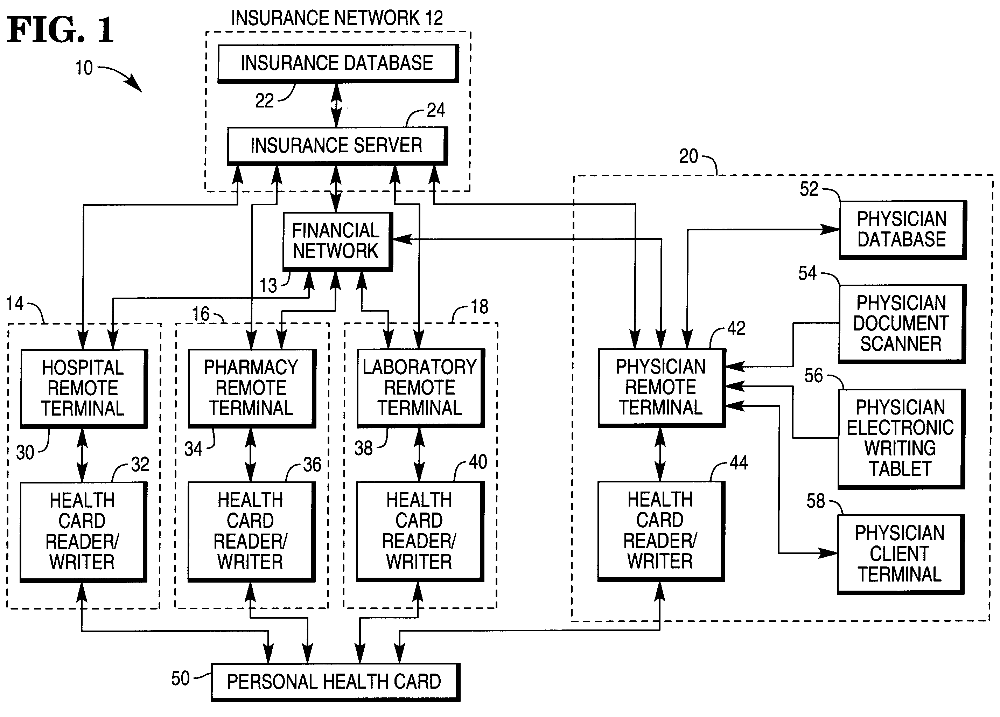

Referring now to Fig. 1, remote access medical network 10 includes insurance network 12, financial network 13, and remote access terminal systems 14, 16, 18, and 20.

Insurance network 12 includes insurance database 22 and insurance server 24. Insurance database 22 stores billing and payment information about patients who have insurance contracts with the insurance company associated with insurance network 12. Insurance server 24 processes payment requests from remote access terminal systems 14-20.

Remote access medical network 10 may include many insurance networks 12, one for each member insurance company.

Financial network 13 provides electronic funds transfer from insurance network 12 to the account of a health care service provider within a bank within network 13.

Remote access medical network 10 may include many financial networks 13.

Remote access terminal systems 14-20 are linked to each insurance network 12 and to each financial network 13, preferably through phone lines and modems. Remote access terminal system 14 includes hospital remote terminal 30 and health card reader/writer 32. Remote access terminal system 16 includes pharmacy remote terminal 34 and health card reader/writer 36. Remote access terminal system 18 includes laboratory remote terminal 38 and health card reader/writer 40. Remote access terminal system 20 includes physician remote terminal 42 and health card reader/writer 44.

Hospital remote terminal 30 transmits a patient's hospital bills to insurance network 12. Pharmacy remote terminal 34 transmits a patient's pharmacy bills to insurance network 12. Laboratory remote terminal 38 transmits a patient's laboratory bills to insurance network 12. Physician remote terminal 42 transmits a patient's physician bills to insurance network 12.

Health care reader/writers 32, 36, 40, and 44 read a patient's billing and health care information from personal health card 50 carried by the patient. This information may include the patient's deductible or co-payment amount under the patient's insurance coverage. It may also include laboratory tests to be completed by the patient and prescriptions to be filled by the patient. Health care reader/writers 32, 36, 40, and 44 also update the health information stored on card 50. Personal health card 50 is preferably a SMART card. Thus, the patient may complete payment and update the information on card 50 at the same time, without any paper forms to fill out and process.

Remote access terminal system 20 may also include physician database 52, document scanner 54, electronic writing tablet 56, and physician client terminal 58.

Physician database 52 stores medical information about the physician's patients for use by the physician. Document scanner 54 is used to scan forms and other paperwork into physician database 52. Electronic writing tablet 56 is used to enter handwritten notes by the physician into physician database 52. Finally, remote terminal 58 allows other users in the physician's office to process patient information.

While only remote access terminal systems 14-20 are shown, the present invention envisions additional remote access terminal systems for other health care service providers. Security is provided by requiring each remote access terminal system to transmit a patient's identification information and a patient's personal identification number (PIN) to insurance network 12. This information is preferably automatically transmitted when the patient's personal health card 50 is read by any of health card reader/writers 32, 36, 40, and 44.

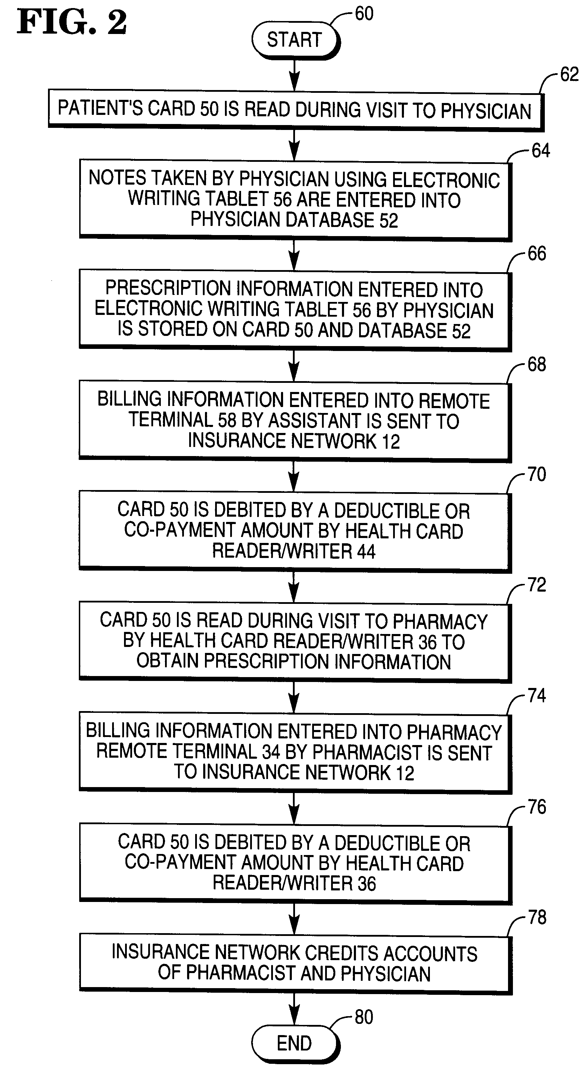

Turning now to Fig. 2, an example illustrating the operation of remote access medical network 10 is shown, beginning with START 60.

In step 62, a patient's card 50 is read by health card reader/writer 44 during the patient's visit.

In step 64, notes taken by the physician using electronic writing tablet 56 are entered into physician database 52.

In step 66, prescription information entered into electronic writing tablet 56 by the physician is stored on card 50 and in physician database 52 under the patient's file.

In step 68, billing information entered into remote terminal 58 by a clerical assistant or nurse is sent to insurance network 12.

In step 70, card 50 is debited by a deductible or co-payment amount by health card reader/writer 44.

In step 72, card 50 is read during a visit to the pharmacy by health card reader/writer 36 to obtain the prescription information from card 50.

In step 74, billing information entered into pharmacy remote terminal 34 by a pharmacist is sent to insurance network 12.

In step 76, card 50 is debited by a deductible or co-payment amount by health card reader/writer 36.

In step 78, insurance network 12 credits the bank accounts of the pharmacist and physician through electronic funds transfer.

In step 80, the method ends.

Similar events would occur upon a trip by the patient to a hospital or laboratory.

## Claims

1. A data processing network for processing medical transactions, characterized by an insurance claim processing netowrk (12) for processing an insurance claim; a remote terminal (30, 34, 38, 42) located at a health care service provider for sending said insurance claim to said insurance claim processing network (12); and a card reader (32, 36, 40, 44) coupled to said remote terminal (30, 34, 38, 42), adapted to reaad information about a patient from a card (50) for use in said insurance claim.

2. A data processing network according to claim 1 characterized in that said insurance claim processing network (12) includes a server (24) adapted to communicate with said remote terminal (30, 34, 38, 42), and a database (22) coupled to said server (24) and adapted to store information about patients.

3. A data processing network according to claim 1 or claim 2, characterized in that said card reader (32, 36, 40, 44) is further adapted to read payment information from said card (50) for use in completing payment to th health care service provider.

4. A data processing network according to any one of claims 1 to 3, characterized in that said card reader (32, 36, 40, 44) is further adapted to write information about the service provided to the patient by the service provider on said card (50).

5. A data processing network according to any one of the preceding claims, characterized in that said card (50) is a smart card.

6. A data processing network according to any one of the preceding claims, characterized in that a further terminal (58) is coupled to said remote terminal (42).

7. A data processing network according to any one of the preceding claims, characterized in that a database (52) for storing patient records is coupled to said remote terminal (42).

8. A data processing system according to any one of the preceding claims, characterized in that an electronic writing tablet (56) is coupled to said remote terminal (42).
